# EUROPEAN PATENT APPLICATION

(11) **EP 4 710 876 A1**
(43) Date of publication of application: **18.03.2026**
(21) Application number: 25199681.5
(22) Date of filing: 02.09.2025
(51) Int. Cl.: A61B 17/70

(54) **SCOLIOSIS CORRECTION DEVICE**

(30) Priority: 11.09.2024 CN 202411270417
(71) Applicant: The First Affiliated Hospital of Sun Yat-sen University, Guangzhou, Guangdong 510080 (CN)
(72) Inventor: Su, Peiqiang, Guangzhou City (CN); Zhou, Taifeng, Guangzhou City (CN); Liao, Zhiheng, Guangzhou City (CN); Zhang, Baolin, Guangzhou City (CN); Chen, Gengjia, Guangzhou City (CN); Fang, Peihang, Guangzhou City (CN)
(74) Representative: Monteiro Alves, Inês

(57) **Abstract**

The present invention relates to the technical field of medical devices and provides a scoliosis correction device, comprising a plurality of transpedicular screws and a flexible orthopedic rod, wherein the plurality of transpedicular screws jointly fix the flexible orthopedic rod comprising a plurality of rod segments and a plurality of connecting members. The plurality of rod segments are movably connected end to end through the plurality of connecting members, and an angle formed by adjacent rod segments ranges from 160° to 180°. By arranging the plurality of movably connected connecting members between the rod segments to form the flexible orthopedic rod, the spine can retain a certain range of natural motion and has a rotational freedom of motion while being corrected in virtue of the flexible orthopedic rod, thereby allowing patients to preserve a certain bending ability. Due to the flexibility of the flexible orthopedic rod between vertebrae in the spine, the spine can retain a certain degree of motion freedom during the correction process, and the patient's ability to move in daily life can be preserved, thereby better completing activities such as bending and turning.

## Description

### TECHNICAL FIELD

The present invention relates to the technical field of medical devices, and in particular to a scoliosis correction device.

### BACKGROUND

Scoliosis is a three-dimensional deformity of the spine, including abnormal sequences in the coronal, sagittal and axial planes. In clinical practice, severity levels of scoliosis can be diagnosed by measuring a Cobb angle through X-rays. When the Cobb angle is in a range of from 10° to 40°, mild scoliosis is diagnosed and non-surgical treatment is recommended; when the Cobb angle is in a range of from 40° to 60°, moderate scoliosis is diagnosed and surgical treatment is recommended; when the Cobb angle is in a range of from 60° to 80°, moderate-to-severe scoliosis is diagnosed and surgical treatment is recommended; when the Cobb angle is greater than 80°, severe scoliosis is diagnosed and surgical treatment is recommended. Scoliosis may cause a spinal deformity, a hunchback, uneven shoulders and backs, a chest deformity, a pelvic tilt, a limb length discrepancy and/or a reduced height, which seriously affect a patient's appearance and cause pain in muscles and joints. In some severe cases, the scoliosis may lead to a reduction in the patient's thoracic volume and even a compression of the heart and/or lungs, resulting in abnormalities in cardiopulmonary development and function and thus a significantly lower motor ability compared to their peers.

In clinical practice, a spinal fusion surgery is usually used to fundamentally treat scoliosis, but a small number of scoliosis patients may undergo non-fusion surgeries for intervention and treatment before the spinal fusion surgery. Non-fusion surgeries include magnetically controlled growing rod (MCGR) procedure, implant-mediated guided growth (IMGG) and anterior vertebral body tethering (AVBT). The MCGR procedure uses a magnetically controlled self-adjusting rod to act as an internal scaffold for straightening the spine. The rod is designed to have two multi-axial joints that allow for a greater motion range; at the same time, the self-adjusting rod allows for additional postoperative correction over time and can accommodate further growth, making it possible to treat both skeletally immature and skeletally mature adolescent patients. The IMGG is specifically designed to treat skeletally immature children under 10 years old, and it not only can correct deformities, but also can maintain the correction over time, thereby minimizing the need for lengthening surgeries repeated at regular intervals. In addition, in the IMGG, a structural rod is fixed by unique non-locking fixation screws at its proximal and distal ends. Such a special feature allows the rod to slide over the screw heads when the child's spine grows, thereby continuously correcting the spinal deformity. The AVBT involves a minimally invasive approach for placement of screws and a tether along the outer portion of the spinal curvature and allows the tether to lengthen as the child grows.

It is true that a small number of scoliosis patients can be treated to a certain extent through non-fusion surgeries in the early stage of scoliosis. However, the above non-fusion surgeries all use metal rods to straighten the spine, which will restrict the natural left-right motion of the spine and make it difficult for the patient to bend the spine. More importantly, a vast majority of scoliosis patients will have to undergo a fusion surgery eventually to achieve a fundamental therapeutic effect. Fusion surgeries include but are not limited to a posterior fusion with or without instrumentation, a combined anterior-posterior fusion, combined anterior-posterior convex hemiepiphysis and hemivertebral resection. All of these surgeries will make the patient's spine fixed and stiff, making the spine difficult to move. Further, there may also be postoperative complications, such as non-fusion of bone graft and kyphosis at the fusion site. Therefore, there is still a lack of a device in clinical practice that can truly save scoliosis patients from fusion surgeries and allow the spine to move.

Therefore, to solve the technical problems in the prior art, it is urgent for those skilled in the art to provide a scoliosis correction device.

### SUMMARY

The purpose of the present invention is to provide a scoliosis correction device . Through adopting the present invention, the spine can retain a certain range of natural motion and has a rotational freedom of motion, thereby allowing patients to preserve a certain bending ability. To achieve the above purpose, the present invention adopts the following technical solution: a scoliosis correction device, comprising a plurality of transpedicular screws and a flexible orthopedic rod, wherein the plurality of transpedicular screws jointly fix the flexible orthopedic rod, the flexible orthopedic rod comprises a plurality of rod segments and a plurality of connecting members; the plurality of rod segments are movably connected end to end through the plurality of connecting members, and an angle formed by adjacent rod segments is in a range of from 160° to 180°.

**In** another preferred embodiment, the connecting members each comprise a hinge housing and two first ball-headed rod, wherein both ends of the hinge housing are respectively provided with a spherical groove or a first spherical cavity matched with the first ball-headed rod; and the hinge housing is connected to two adjacent rod segments through the two first ball-headed rods, so that the two adjacent rod segments are movably connected end to end.

**In** another preferred embodiment, the plurality of transpedicular screws each comprise a screw body and a screw cap, wherein the screw cap is arranged on the screw body and provided with a through hole sized to fit the hinge housing, and the hinge housing is located in the through hole.

**In** another preferred embodiment, the transpedicular screws each comprise a screw body, a screw cap and a second ball-headed rod, wherein the hinge housing is further provided with a second spherical cavity, one end of the second ball-headed rod is provided with a ball-headed matching with the second spherical cavity, while another end of the second ball-headed rod is arranged on the screw cap, and the screw cap is located on the screw body.

**In** another preferred embodiment, the first ball-headed rod and the screw body are perpendicular to each other.

**In** another preferred embodiment, the connecting members each comprise a hinge housing and a first ball-headed rod, wherein the hinge housing is provided with a first spherical cavity, the first ball-headed rod is matched with the first spherical cavity, and the first ball-headed rod and the hinge housing are respectively arranged at corresponding ends of two adj acent rod segments, so that the two adjacent rod segments are movably connected end to end.

**In** another preferred embodiment, the hinge housing is provided with a limit slot, the limit slot is in communication with first spherical cavity to define a rotation range of the first ball-headed rod.

**In** another preferred embodiment, a rotation angle of the first ball-headed rod defined by the limit slot is in a range of from 0° to 20°.

**In** another preferred embodiment, the connecting members each comprise a pin seat and a pin, wherein the pin seat is provided with a pin hole for installing the pin, the pin is located in the pin hole, a plurality of the connecting members are provided between two adjacent transpedicular screws, and two adjacent pins are perpendicular to each other.

**In** another preferred embodiment, the transpedicular screws each comprise a screw body, a screw seat and a fastening stud, wherein the screw body is rotatably connected to an inner side of a bottom portion of the screw seat, a threaded section of the screw body is located below the screw seat, through grooves are symmetrically provided on both sides of the screw seat, and a thread groove is provided on an inner side of a top portion of the screw seat, the fastening stud is threadedly connected to the thread groove, and the rod segments are clamped between the screw body and the fastening stud.

**In** another preferred embodiment, the rod segment is a telescopic rod segment.

**In** another preferred embodiment, the telescopic rod segment is provided with an outer joint rod, an inner joint rod, and a locking member for locking the relative positions of the inner joint rod and the outer joint rod, wherein the inner joint rod is movably sleeved inside the outer joint rod, and the locking member is movably assembled on the outer joint rod.

**In** another preferred embodiment, the surface of the locking member is provided with convex teeth.

**In** another preferred embodiment, an outer surface of one end of the inner joint rod is integrally connected with ratchet teeth for interacting with the convex teeth, wherein one end connected to the ratchet teeth is defined as a first working end, the first working end is telescopically sleeved inside the outer joint rod, the locking member is movably assembled on the outer joint rod, and the convex teeth are engaged with the ratchet teeth.

**In** another preferred embodiment, the outer joint rod is provided with a second through hole for exposing the ratchet teeth and an assembly area for assembling with the locking member, wherein the second through hole is located inside the assembly area, the locking member is movably assembled in the assembly area, and the ratchet teeth extend to the assembly area through the second through hole. When adjusting the length of the telescopic rod segment, an elastic pawl is compressed, so that the convex teeth are separated from the ratchet teeth and the position of the inner joint rod is adjusted. After the elastic pawl is restored, the convex teeth are engaged with the ratchet teeth, thereby locking the positions of the inner joint rod and the outer joint rod.

**In** another preferred embodiment, the locking member is a fine-teeth cam, and the fine-teeth cam is rotatably connected to the assembly area through a shaft. When the length of the telescopic rod segment is adjusted, the fine-teeth cam rotates, so that the convex teeth are separated from the ratchet teeth and the position of the inner joint rod is adjusted. After the fine-teeth cam is restored, the convex teeth are engaged with the ratchet teeth, thereby locking the positions of the inner joint rod and the outer joint rod.

**In** another preferred embodiment, the locking member is a locking knob, the locking knob is sleeved on an outer surface of the outer joint rod, and the locking knob is in threaded connection with the outer joint rod.

**In** another preferred embodiment, an outer surface of one end of the outer joint rod is integrally connected with an external thread for interacting with the locking knob, wherein one end connected to the external thread is defined as a second working end, and the inner joint rod is telescopically sleeved inside the second working end.

**In** another preferred embodiment, a plurality of longitudinal slits are arranged at an end of the second working end, and an inner surface of the second working end is integrally connected with teeth.

In another preferred embodiment, a plurality of longitudinal slits are arranged at an end of the second working end, an inner surface of the second working end is integrally connected with teeth, and the ratchet teeth are engaged with the teeth.

In another preferred embodiment, when adjusting the length of the telescopic rod segment, the locking knob is rotated to the outside of the second working end, so that the position of the inner joint rod is adjusted. When the locking knob is rotated to an end of the second working end, the positions of the inner joint rod and the outer joint rod are locked.

In another preferred embodiment, the connecting member is provided with a lantern ring and a rotating ring, wherein the rotating ring is rotatably assembled inside the lantern ring, and the transpedicular screw penetrates through the rotating ring.

In another preferred embodiment, the lantern ring is provided with an opening, and the rotating ring is a closed ring.

Compared with the prior art, the scoliosis correction devices in the embodiments of the present invention have the following beneficial effects. By arranging the plurality of movably connected connecting members between the plurality of rod segments to form the flexible orthopedic rod, the spine can retain a certain range of natural motion and has a rotational freedom of motion while being corrected in virtue of the flexible orthopedic rod, allowing patients to preserve a certain bending ability. Since the flexible orthopedic rod has a certain degree of freedom of motion between multiple vertebrae of the spine, the scoliosis correction device can more accurately adapt to the patient's spinal curvature. The spine can retain a certain degree of freedom of motion during the scoliosis correction process, while the patient will retain the motor ability in daily life and can better complete activities, such as bending and turning.

### BRIEF DESCRIPTION OF THE DRAWINGS

Figure 1 is a first perspective view of a scoliosis correction device provided by the present invention;
Figure 2 is a first bottom view of the scoliosis correction device provided by the present invention;
Figure 3 is a side view of the scoliosis correction device provided by the present invention;
Figure 4 is a second bottom view of the scoliosis correction device provided by the present invention;
Figure 5 is a second perspective view of the scoliosis correction device provided by the present invention, further illustrating a disassembled flexible orthopedic rod;
Figure 6 is a third perspective view of the scoliosis correction device provided by the present invention, further illustrating a disassembled flexible orthopedic rod;
Figure 7 is a fourth perspective view of the scoliosis correction device provided by the present invention;
Figure 8 shows a fifth perspective view of the scoliosis correction device provided by the present invention, further illustrating a disassembled part;
Figure 9 is a schematic diagram illustrating an exemplary structure when the scoliosis correction device in embodiment 2 and the spine are assembled;
Figure 10 is a schematic diagram illustrating an exemplary structure of the scoliosis correction device in embodiment 2;
Figure 11 is a schematic diagram illustrating an exemplary structure of flexible orthopedic rod in embodiment 2;
Figure 12 is a schematic diagram illustrating a sectional view in the "A-A" direction of Figure 11;
Figure 13 is a schematic diagram illustrating an exemplary structure of the locking member in Figure 10;
Figure 14 is a schematic diagram illustrating an exemplary structure when the scoliosis correction device in embodiment 3 and the spine are assembled;
Figure 15 is a schematic diagram illustrating a partial exemplary structure of the scoliosis correction device in embodiment 3;
Figure 16 is a schematic diagram illustrating a partial sectional view of the scoliosis correction device in embodiment 3;
Figure 17 is a schematic diagram illustrating an exemplary structure of the locking member in Figure 15;
Figure 18 is a schematic diagram illustrating an exemplary structure when the scoliosis correction device in embodiment 4 and the spine are assembled;
Figure 19 is a schematic diagram illustrating a partial exemplary structure of the scoliosis correction device in embodiment 4;
Figure 20 is a schematic diagram illustrating a partial sectional view of the scoliosis correction device in embodiment 4;
Figure 21 is a schematic diagram illustrating an exemplary structure of the scoliosis correction device in embodiment 5; and
Figure 22 is a schematic diagram illustrating a partial sectional view of the scoliosis correction device in embodiment 5;

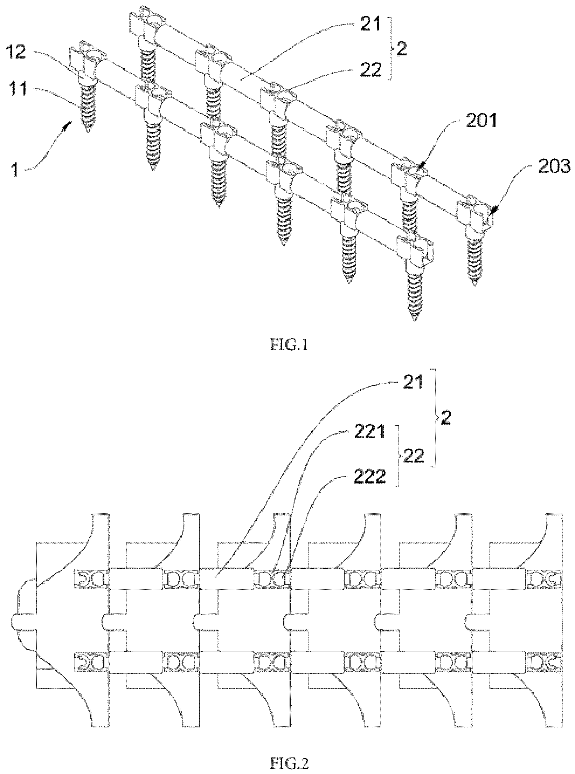

In Figures 1-22: 1-Transpedicular Screw, 11-Screw Body, 12-Screw Cap, 13-Second Ball-headed Rod, 14-Screw Seat, 15-Fastening Stud, 101-Through Hole,
2-Flexible Orthopedic Rod, 21-Rod Segment, 211-Outer Joint Rod, 2111-Assembly Area, 2112-Slit, 2113-Tooth, 212-Inner Joint Rod, 2121-Ratchet Tooth, 213-Locking Member, 2131-Convex Tooth, 2132-Pawl Main Body, 2133-Elastic Piece, 22-Connecting Member, 221-Hinge Housing, 222-First Ball-headed Rod, 223-Pin Seat, 224-Pin, 201-Spherical Groove, 202-First Spherical Cavity, 203-Limit Slot, 225-Lantern Ring, 226-Rotating Ring, 227-Third Ball-headed Rod, 228-Concave Body, 229-Universal Spherical Shell.

### DETAILED DESCRIPTION

Detailed embodiments are combined hereinafter to further elaborate the technical solution of the present invention. For ease of description, one end of the scoliosis correction device close to a user end during use is defined as a front end, and one end away from the user end is defined as a rear end.

As shown in Figures 1 to 8, in an embodiment of the present invention, a scoliosis correction device is provided, comprising a plurality of transpedicular screws 1 and a flexible orthopedic rod 2, wherein the plurality of transpedicular screws 1 jointly fix the flexible orthopedic rod 2, and the flexible orthopedic rod 2 comprises a plurality of rod segments and a plurality of connecting members 22; the plurality of rod segments 21 are movably connected end to end by the plurality of connecting members 22, and an angle A formed by adjacent rod segments 21 is in a range of from 160° to 180°.

**In** the scoliosis correction device of the present invention, by arranging the plurality of movably connected connecting members 22 between the plurality of rod segments 21 to constitute the flexible orthopedic rod 2, the spine can retain a certain range of natural motion and has a rotational freedom of motion while being corrected in virtue of the flexible orthopedic rod 2, allowing patients to preserve a certain bending ability.

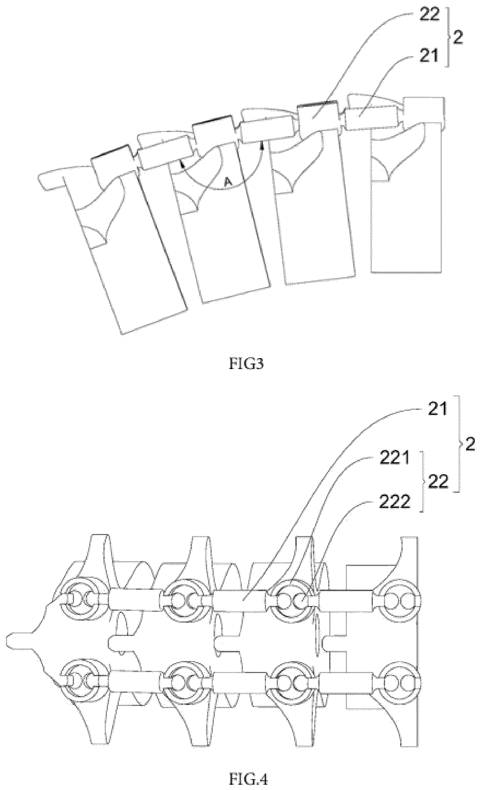

Since the flexible orthopedic rod 2 has a certain degree of freedom of motion between multiple vertebrae of the spine, the scoliosis correction device can more accurately adapt to the patient's spinal curvature. The spine can retain a certain degree of freedom of motion during the scoliosis correction process, while the patient will retain the motor ability in daily life and can better complete activities, such as bending and turning, thereby improving the quality of life. By allowing the flexible orthopedic rod 2 to be constituted by multiple rod segments 21 which are interconnected end to end via a plurality of connecting members 22, as shown in Figure 3, the angle A formed between adjacent rod segments 21 is in a range of from 160° to 180°, so that the spine can retain a certain degree of rotational freedom between adjacent vertebrae within a certain spatial range and can move naturally within a certain range, while maintaining the correction effect. Thus, the scoliosis correction device of the present invention reduce the stiffness brought by traditional fixation devices and can better adapt to natural motions and changes of the patient's body, thereby providing greater comfort and adaptability. Therefore, the flexible orthopedic rod 2 enables the scoliosis correction device to adapt to growth and changes of the spine while ensuring the correction effect, thereby being conducive to achieving a better correction effect in long-term use.

As shown in Figures 1 and 2, in an optional embodiment of the present invention, the connecting members 22 each include a hinge housing 221 and two first ball-headed rods 222, wherein both ends of the hinge housing 221 are respectively provided with a spherical groove 201 or a first spherical cavity 202, which is matched with the first ball-headed rod 222; the hinge housing 221 is arranged at corresponding ends of two adjacent rod segments 21 through two first ball-headed rods 222, so that the two adjacent rod segments 21 are movably connected end to end. The interaction between the hinge housing 221 and the first ball-headed rods 222 allows the connecting members 22 to provide flexible degrees of freedom of motion. At the same time, the scoliosis correction device is allowed to be fine-tuned and limited at different angles and directions, so that the scoliosis correction is more accurate and the device can adapt to the natural curvature of the individual spine.

The interaction between the first ball-headed rod 222 and the spherical groove 201 or the first spherical cavity 202 reduces friction and hard contact points at the connection areas, making the scoliosis correction device more comfortable during use and reducing the potential discomfort caused by traditional rigid connections. Due to the movable structure of the hinge housing 221 and the ball-headed rod, the scoliosis correction device can achieve natural degrees of freedom of motion for the spine during the scoliosis correction process. In this way, patients can retain a certain motor ability, such as bending and turning, during the scoliosis correction process, thereby improving the quality of life. The interaction between the first ball-headed rod 222 and the spherical groove 201 or the first spherical cavity 202 also enables the scoliosis correction device to better adapt to the spinal curvature of individual patients. Further, the connecting member 22 can adaptively adjust the angle formed by the adjacent rod segments 21 according to the spinal curvature, and thus can accurately adjust the correction angle, thereby achieving a more effective correction effect.

As shown in Figure 3 and Figure 4, in an optional embodiment of the present invention, the connecting members 22 each comprise a hinge housing 221 and two first ball-headed rods 222, and the hinge housing 221 is provided with a first spherical cavity 202; ball-headeds of the two first ball-headed rods 222 are both located in the first spherical cavity 202, and the two first ball-headed rods 222 are respectively arranged at corresponding ends of two adjacent rod segments 21, so that the two adjacent rod segments 21 are movably connected end to end.

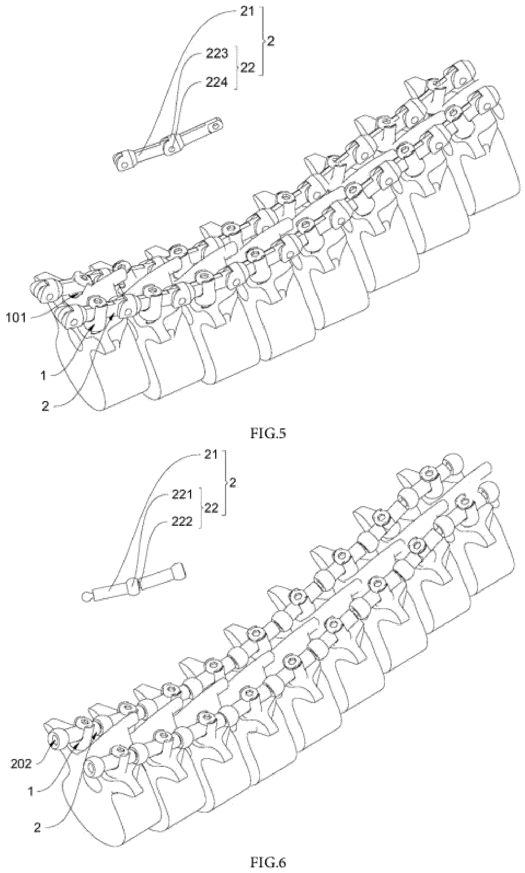

As shown in Figure 1 and Figure 5, in an optional embodiment of the present invention, the plurality of transpedicular screws each 1 include a screw body 11 and a screw cap 12, wherein the screw cap 12 is arranged on the screw body 11 and provided with a through hole 101 sized to fit the hinge housing 221, and the hinge housing 221 is located in the through hole 101.

By matching the through hole 101 of the screw cap 12 with the hinge housing 221 in sizes, an accurate connection between the transpedicular screw and the hinge housing 221 is ensured, thereby reducing installation errors of the flexible orthopedic rod 2 and improving the stability and effectiveness of the scoliosis correction device. The screw cap 12 ensures a more secure fixation of the transpedicular screw to the hinge housing 221, thereby increasing the stability of the flexible orthopedic rod 2. Such connection is also conducive to maintaining the correction effect and preventing looseness or displacement of the flexible orthopedic rod 2, which may affect the correction effect during the scoliosis correction process. Further, the through hole 101 can ensure an accurate position of the hinge housing 221 in the screw cap 12, thereby avoiding possible failure or damage caused by improper connection and improving safety of the scoliosis correction device during use.

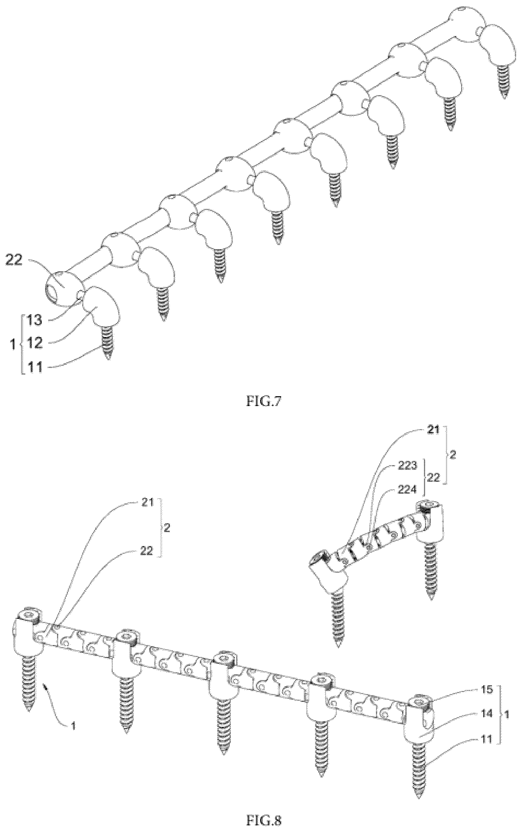

As shown in Figure 7, in an optional embodiment of the present invention, the transpedicular screws 1 each comprise a screw body 11, a screw cap 12 and a second ball-headed rod 13; the hinge housing 221 is also provided with a second spherical cavity; one end of the second ball-headed rod 13 is provided with a ball-headed matching with the second spherical cavity, while the other end of the second ball-headed rod 13 is arranged on the screw cap 12, which is further arranged on the screw body 11.

The interaction between the second ball-headed rod 13 and the second spherical cavity allows the second ball-headed rod 13 to provide greater joint flexibility. The presence of the ball-headed in the second ball-headed rod 13 allows the transpedicular screw to be adjusted at different angles and directions, thereby improving the flexibility of scoliosis correction and adapting to complex conditions of natural curvature of the spine. The ball-headed equipped at one end of the second ball-headed rod 13 can be accurately embedded in the second spherical cavity of the hinge housing 221, which is beneficial for the connection and fixation of the flexible orthopedic rod 2 by the transpedicular screws 1. The interaction between the screw cap 12 and the screw body 11, in combination with the second ball-headed rod 13, increases the stability of the flexible orthopedic rod 2. Moreover, the connection between the screw cap 12 and the second ball-headed rod 13 in the transpedicular screw allows the flexible orthopedic rod 2 to be more firmly fixed on the spine, reducing the risk of displacement or looseness of the flexible orthopedic rod 2 caused by motions or load changes.

As shown in Figure 7, in an optional embodiment of the present invention, the first ball-headed rod 222 is perpendicular to the screw body 11. By making the first ball-headed rod 222 perpendicular to the screw body 11, the scoliosis correction device can distribute force more evenly when bearing loads from the spine. Such vertical configuration also makes the force transmission path more direct and is conducive to reducing unnecessary lateral stress, thereby enhancing the stability of the scoliosis correction device and its support force for the spine during the scoliosis correction process. In addition, the vertical configuration of the first ball-headed rod 222 and the screw body 11 enables the scoliosis correction device to maintain a high structural integrity during use, thereby reducing the risk of deformation or damage of the scoliosis correction device due to uneven force and thus improving the long-term stability of the scoliosis correction device.

During an operation, the vertical configuration simplifies the installation and layout of the scoliosis correction device. Since the relative position between the screw body 11 and the first ball-headed rod 222 remains fixed, medical staff can more intuitively position and install the flexible orthopedic rod 2 by virtue of the first ball-headed rod 222, thereby reducing the difficulty and time of the operation. Additionally, by keeping the first ball-headed rod 222 perpendicular to the screw body 11, the distance between the flexible orthopedic rod 2 and the spine can be reduced, so that the flexible orthopedic rod 2 can be more closely fitted with the spine at identical screw body 11 heights, which facilitates the implanting of the flexible orthopedic rod 2 in a confined space.

As shown in Figure 6, in an optional embodiment of the present invention, the connecting members 22 each comprise a hinge housing 221 and a first ball-headed rod 222, wherein the hinge housing 221 is provided with a first spherical cavity 202, which is adapted to the first ball-headed rod 222; the first ball-headed rod 222 and the hinge housing 221 are respectively arranged at corresponding ends of two adjacent rod segments 21, so that the two adjacent rod segments 21 are movably connected end to end.

The interaction between the first spherical cavity 202 in the hinge housing 221 and the first ball-headed rod 222 enables the rod segments 21 at both ends of the connecting member 22 to rotate in multiple directions and provides a larger range of motion and flexibility for the flexible orthopedic rod 2, so that the flexible orthopedic rod 2 can better adapt to the complex curvature of the spine, thereby improving the correction effect. Besides, thanks to the spherical interaction between the first ball-headed rod 222 and the hinge housing 221, the installation and adjustment of the flexible orthopedic rod 2 becomes easier. Medical staff can more easily position and fix the connecting members 22, thereby saving surgical time and reducing the complexity of the operation.

The spherical interaction between the hinge housing 221 and the first ball-headed rod 222 provides a firm connection, thereby reducing the loss of correction effect due to looseness or displacement of components of the scoliosis correction device, increasing the long-term stability and durability of the flexible orthopedic rod 2, and improving the durability of the correction effect. The spherical interaction also makes the force transmission more uniform and stable and reduces the local stress concentration phenomenon, thus the flexible orthopedic rod 2 can distribute the force more evenly when bearing loads from the spine, thereby improving the support effect on the spine during the scoliosis correction process. Moreover, the spherical interaction between the first spherical cavity 202 and the first ball-headed rod 222 allows the rod segments 21 to be fine-tuned at different angles and directions, thereby increasing the adaptability of the flexible orthopedic rod 2 to different spinal morphologies. This flexibility enables the scoliosis correction device to better meet individualized correction needs, thereby improving the pertinence and accuracy of the correction effect.

**In** an optional embodiment of the present invention, the hinge housing 221 is provided with a limit slot 203, which is in communication with the first spherical cavity 202 to define a rotation range of the first ball-headed rod 222.

By allowing the limit slot 203 and the first spherical cavity 202 to be communicated with each other, the rotation range of the first ball-headed rod 222 can be accurately controlled, ensuring that the first ball-headed rod 222 moves within a preset range. Such configuration is conducive to achieving more accurate scoliosis correction and avoids unstable correction effects caused by excessive rotation. By defining the rotation range of the first ball-headed rod 222, the limit slot 203 can effectively prevent the connecting members 22 from excessive motion or loss of control during the scoliosis correction process, which is beneficial for protecting the spine from applying an excessive force and reducing potential injury risks. At the same time, the spinal range of motion will not be too large, thereby ensuring the correction effect.

The arrangement of the limit slot 203 in the hinge housing 221 increases the reliability of the flexible orthopedic rod 2 and ensures that the flexible orthopedic rod 2 works stably within the preset range of motion, thereby improving the stability of the overall correction effect. The fluctuation of the correction effect, caused by excessive or unpredictable motion of the flexible orthopedic rod 2, is reduced. By defining a clear rotation range, the limit slot 203 simplifies the adjustment process of the flexible orthopedic rod 2. By replacing the connecting member 22 with a different limit range, the medical staff can more easily define the range of motion of the flexible orthopedic rod 2, thereby reducing complexity of adjustment and possibility of operational errors. Additionally, the connecting member 22 with a limited rotation range can avoid excessive pressure or discomfort inflicted on the patient's spine by the scoliosis correction device, thereby making the scoliosis correction device more comfortable during use. The connecting member 22 also can mitigate postoperative discomfort or pain potentially caused by uncontrolled motions. Moreover, the connecting member 22 with a limit slot 203 increases the safety of the scoliosis correction device. Specifically, by controlling the rotation range, the connecting member 22 reduces the potential risk caused by unexpected motions or mechanical failure, thereby providing higher-level safety assurance.

Specifically, the connecting members 22 each are detachably connected to the rod segments 21. There are various connecting members 22 that restrict the rotation ranges of adjacent rod segments 21. The various connecting members 22 achieve different rotation ranges, through limit slots 203 with different sizes. According to the patient's scoliosis condition, the connecting members 22 with corresponding limited rotation ranges are selected.

In an optional embodiment of the present invention, a rotation angle of the first ball-headed rod 222 defined by the limit slot is in a range of from 0° to 20°. By defining the rotation angle in a range of from 0° to 20°, corrective degrees of freedom applied to the spine by the scoliosis correction device can be precisely controlled, which is conducive to performing precise correction according to specific needs of the individuals, optimizing the correction effect and ensuring that the correction effect is not too strong or too weak. Defining the rotation angle in a range of from 0° to 20° also effectively prevents overcorrection during the scoliosis correction process. Overcorrection may cause discomfort or further spinal problems to patients, but the limitation of the limit slot 203 can ensure that the scoliosis correction process is carried out within a safe and effective range. A clear and small rotation angle range can reduce accidental over-rotation, thereby reducing the risk of injuries to the spine during the scoliosis correction process. Safe limitations of the rotation angle can effectively prevent potential injuries caused by uncontrolled angles. Defining the rotation angle in a range of from 0° to 20° can ensure that the scoliosis correction device operates within a small, controllable and stable range, which is conducive to maintaining the stability and reliability of the correction effect.

As shown in Figure 8, in an optional embodiment of the present invention, the connecting members 22 each comprise a pin seat 223 and a pin 224, wherein the pin seat 223 is provided with a pin hole for installing the pin 224, and the pin 224 is located in the pin hole; a plurality of connecting members 22 are provided between two adjacent transpedicular screws 1, and two adjacent pins 224 are perpendicular to each other. By providing a pin hole in the pin seat 223 for installing the pin 224, a tight fit between the pin 224 and the pin seat 223 can be ensured. The precise installation of the pin 224 provides enhanced connection firmness, thereby reducing the risk of unstable correction effects, caused by looseness or detachment of the connecting components.

The arrangement of the plurality of connecting members 22 and the arrangement of two adjacent pins 224 perpendicular to each other allow the rod segments 21 to move in two different directions, so that the flexible orthopedic rod 2 can adapt to spines with different curvatures, thereby improving the flexibility of the scoliosis correction device. Thus, the scoliosis correction device can be adjusted in different correction schemes and is suitable for various scoliosis conditions. Additionally, the interaction of the pin 224 and the pin seat 223 makes the assembly and disassembly of the connecting members 22 easier. The assembly and disassembly of the pin 224 are relatively simple, thereby reducing the complexity during the assembly process of the scoliosis correction device and improving the operational efficiency. This is particularly suitable for situations where the scoliosis correction device is required to be adjusted or disassembled frequently.

The connection of the pin 224 and the pin seat 223 can reduce the need for complex connecting components, which is conducive to reducing manufacturing costs. At the same time, standardized pin 224 and pin seat 223 are also beneficial for improving production efficiency, thereby further reducing the cost of the scoliosis correction device. The interaction of the pin 224 and the pin seat 223 can disperse mechanical stress and reduce wear caused by long-term use of the scoliosis correction device or heavy loads born by the device, thereby improving the durability and service life of the scoliosis correction device and ensuring good performance maintained during the long correction process.

As shown in Figure 5 and Figure 8, in an optional embodiment of the present invention, the transpedicular screws 1 each comprise a screw body 11, a screw seat 14 and a fastening stud 15, wherein the screw body 11 is rotatably connected to an inner side of a bottom portion of the screw seat 14, a threaded section of the screw body 11 is located below the screw seat 14; through grooves are symmetrically provided on both sides of the screw seat 14, and a threaded groove is provided on an inner side of a top portion of the screw seat 14; the fastening stud 15 is threadedly connected to the threaded groove, and the rod segments 21 are clamped between the screw body 11 and the fastening stud 15.

Since the screw body 11 is rotatably connected to the inner side of the bottom portion of the screw seat 14, a tight connection between the screw body 11 and the screw seat 14 is ensured, which can effectively improve the fixation stability between the screw body 11 and the vertebra of the spine, prevent looseness or displacement during use, and thus provide a more reliable correction effect. The threaded connection between the threaded groove on the inner side of the top portion of the screw seat 14 and the fastening stud 15 makes the installation process simpler and more efficient. By tightening the fastening stud 15 through threaded adjustment, the rod segments 21 clamped between the screw body 11 and the fastening stud 15 are fixed, thereby ensuring the stability of the flexible orthopedic rod 2 and improving the operational simplicity during the fixation process of the rod segments 21. The clamping arrangement of the rod segments 21 between the screw body 11 and the fastening stud 15 can provide additional support and stability. Therefore, not only the overall strength of the scoliosis correction device is enhanced, but also the correction force is effectively, evenly distributed across the spine, thereby reducing local pressures and improving comfort and effectiveness.

### Embodiment 2

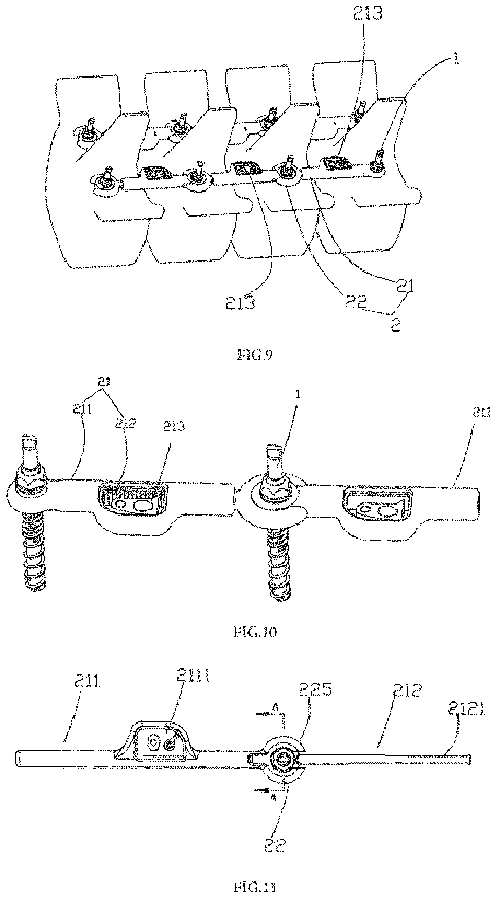

Figures 9-13 show a scoliosis correction device in embodiment 2. In this embodiment, the rod segment 21 is a telescopic rod segment 21. The telescopic rod segment 21 is provided with an outer joint rod 211, an inner joint rod 212 and a locking member 213 for locking the relative positions of the inner joint rod 212 and the outer joint rod 211. The inner joint rod 212 is movably sleeved inside the outer joint rod 211, and the locking member 213 is movably assembled on the outer joint rod 211.

The surface of the locking member 213 is provided with convex teeth 2131. As shown in Figures 10-12, an outer surface of one end of the inner joint rod 212 is integrally connected to ratchet teeth 2121 for interacting with the convex teeth 2131, one end connected to the ratchet teeth 2121 is defined as a first working end, and the first working end is telescopically sleeved inside the outer joint rod 211. The locking member 213 is movably assembled on the outer joint rod 211, and the convex teeth 2131 are engaged with the ratchet teeth 2121.

The outer joint rod 211 is provided with a second through hole (not shown) for exposing the ratchet teeth 2121 and an assembly area 2111 for assembly with the locking member 213. The second through hole is located inside the assembly area 2111, the locking member 213 is movably assembled in the assembly area 2111, and the ratchet teeth 2121 extend to the assembly area 2111 through the second through hole.

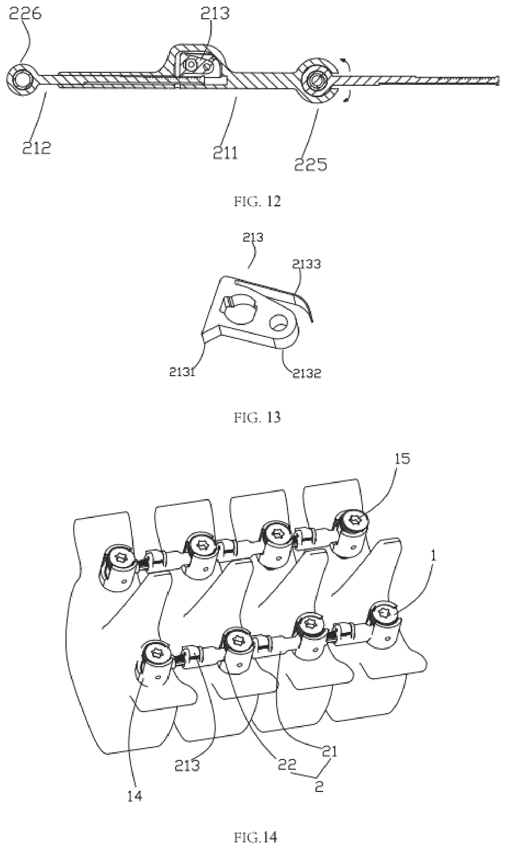

As shown in Figure 13, the locking member 213 is an elastic pawl, and the elastic pawl is movably embedded in the assembly area 2111. The elastic pawl is provided with a pawl main body 2132 and an elastic piece 2133. The convex teeth 2131 and the elastic piece 2133 are respectively integrally connected to the pawl main body 2132. The assembly area 2111 is an assembly groove, and the elastic pawl is embedded inside the assembly groove.

When adjusting the length of the telescopic rod segment 21, the elastic pawl is compressed by the medical staff, so that the convex teeth 2131 are separated from the ratchet teeth 2121 and the position of the inner joint rod 212 is adjusted. After being released, the elastic pawl automatically interacts with the external thread and returns to its original position. At this point, the convex teeth 2131 are engaged with the ratchet teeth 2121, and the positions of the inner joint rod 212 and the outer joint rod 211 are locked.

As shown in Figures 10-12, the connecting member 22 is provided with a lantern ring 225 and a rotating ring 226. The rotating ring 226 is rotatably assembled inside the lantern ring 225, and the transpedicular screw (not shown) penetrates through the rotating ring 226. The lantern ring 225 is provided with an opening, and the rotating ring 226 is a closed ring. When the lantern ring 225 is integrally connected to the outer joint rod 211, the rotating ring 226 is integrally connected to the inner joint rod 212. When the lantern ring 225 is integrally connected to the inner joint rod 212, the rotating ring 226 is integrally connected to the outer joint rod 211. In this embodiment, the lantern ring 225 is integrally connected to the outer joint rod 211, and the rotating ring 226 is integrally connected to the inner joint rod 212. In this embodiment, the connecting member 22 has a simple structure and a small size.

It is worth mentioning that the rotating ring 226 in this embodiment rotates along the opening of the lantern ring 225 by taking the central axis of the transpedicular screw as the center, thereby achieving a certain degree of motion freedom.

Compared with embodiment 1, except for possessing a certain degree of motion freedom, the scoliosis correction device in this embodiment may also extend and retract according to the actual situation of the spine, thereby satisfying the requirements of different situations.

### Embodiment 3

Figures 14-17 show a scoliosis correction device in embodiment 3. In this embodiment, the rod segment 21 is a telescopic rod segment 21. The telescopic rod segment 21 is provided with an outer joint rod 211, an inner joint rod 212 and a locking member 213 for locking the relative positions of the inner joint rod 212 and the outer joint rod 211. The inner joint rod 212 is movably sleeved inside the outer joint rod 211, and the locking member 213 is movably assembled on the outer joint rod 211.

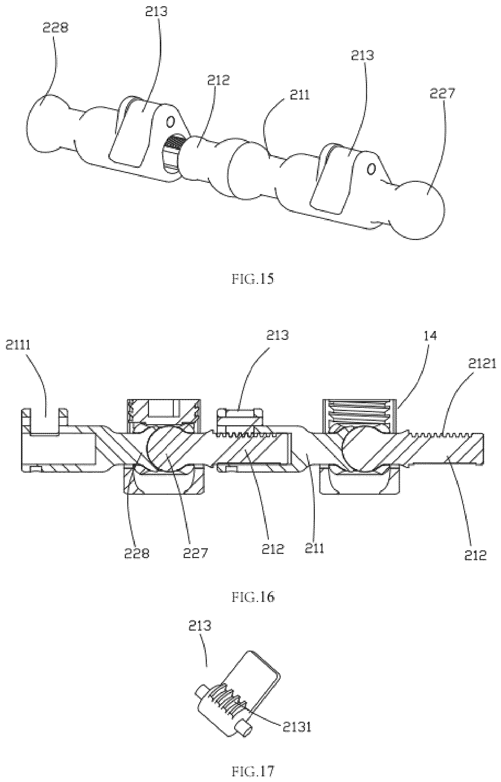

The surface of the locking member 213 is provided with convex teeth 2131. As shown in Figures 15-16, an outer surface of one end of the inner joint rod 212 is integrally connected to ratchet teeth 2121 for interacting with the convex teeth 2131, one end connected to the ratchet teeth 2121 is defined as a first working end, and the first working end is telescopically sleeved inside the outer joint rod 211. The locking member 213 is movably assembled on the outer joint rod 211, and the convex teeth 2131 are engaged with the ratchet teeth 2121.

The outer joint rod 211 is provided with a second through hole (not shown) for exposing the ratchet teeth 2121 and an assembly area 2111 for assembly with the locking member 213. The second through hole is located inside the assembly area 2111, the locking member 213 is movably assembled in the assembly area 2111, and the ratchet teeth 2121 extend to the assembly area 2111 through the second through hole.

As shown in Figure 17, the locking member 213 is a fine-teeth cam. The fine-teeth cam may be rotatably axially assembled in the assembly area 2111. When adjusting the length of the telescopic rod segment 21, the fine-teeth cam is rotated, so that the convex teeth 2131 are separated from the ratchet teeth 2121 and the position of the inner joint rod 212 is adjusted.

When the fine-teeth cam is restored, the convex teeth 2131 are engaged with the ratchet teeth 2121, thereby locking the positions of the inner joint rod 212 and the outer joint rod 211.

The connecting member 22 is provided with a third ball-headed rod 227 and a concave body 228 for interacting with the third ball-headed rod 227. The third ball-headed rod 227 is slidably abutted against the concave body 228. When the concave body 228 is integrally connected to the outer joint rod 211, the third ball-headed rod 227 is integrally connected to the inner joint rod 212. When the concave body 228 is integrally connected to the inner joint rod 212, the third ball-headed rod 227 is integrally connected to the outer joint rod 21. In this embodiment, the concave body 228 is integrally connected to the outer joint rod 211, and the third ball-headed rod 227 is integrally connected to the inner joint rod 212.

It is worth mentioning that, the third ball-headed rod 227 and the concave body 228 form a joint structure, enabling the third ball-headed rod 227 to rotate such that a certain degree of motion freedom is achieved.

The transpedicular screw 1 is provided with a screw seat 14, a fastening stud 15 and a screw body (not shown). One end of the screw body penetrates from a lower portion of the screw seat 14 to the outside, the third ball-headed rod 227 and the concave body 228 are both located inside the screw seat 14, and the fastening stud 15 is in threaded connection with an upper end of the screw seat 14.

Compared with embodiment 1, except for possessing a certain degree of motion freedom, the scoliosis correction device in this embodiment may also extend and retract according to the actual situation of the spine, thereby satisfying the requirements of different situations.

### Embodiment 4

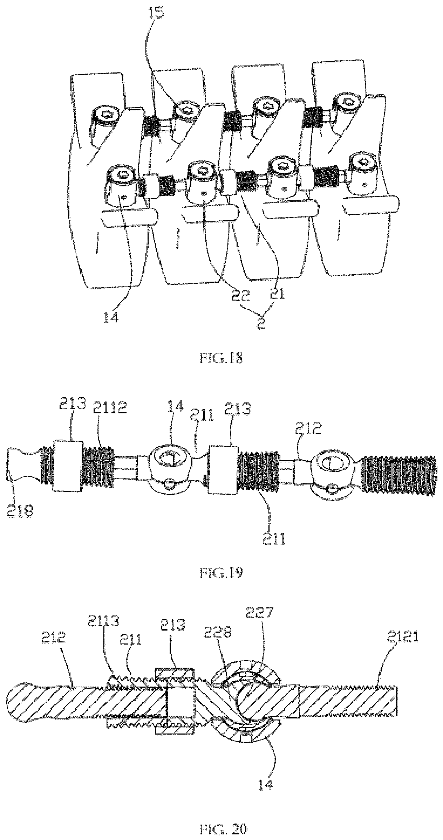

Figures 18-20 show a scoliosis correction device in embodiment 4. In this embodiment, the rod segment 21 is a telescopic rod segment 21. The telescopic rod segment 21 is provided with an outer joint rod 211, an inner joint rod 212 and a locking member 213 for locking the relative positions of the inner joint rod 212 and the outer joint rod 211. The inner joint rod 212 is movably sleeved inside the outer joint rod 211, and the locking member 213 is movably assembled on the outer joint rod 211.

The locking member 213 is a locking knob, the locking knob is sleeved on an outer surface of the outer joint rod 211, and the locking knob is in threaded connection with the outer joint rod 211.

An outer surface of one end of the outer joint rod is integrally connected with an external thread for interacting with the locking knob, wherein one end connected to the external thread is defined as a second working end, and the inner joint rod is telescopically sleeved inside the second working end. A plurality of longitudinal slits are arranged at an end of the second working end, an inner surface of the second working end is integrally connected with teeth 2113, and the the ratchet teeth 2121 are engaged with the teeth 2113.

When adjusting the length of the telescopic rod segment 21, the locking knob is rotated to the outside of the second working end, so that the position of the inner joint rod 212 is adjusted. Subsequently, the locking knob is rotated to the end of the second working end, thereby locking the positions of the inner joint rod 212 and the outer joint rod 211.

It is worth mentioning that, because the end of the second working end is provided with a plurality of slits 2112, when the locking knob is rotated to the outside of the second working end, the inner joint rod 212 is separated from the second working end, allowing the inner joint rod 212 to move in a telescopic manner. After the inner joint rod 212 moves to a proper position, the locking knob is rotated to the end of the second working end, and the slits 2112 of the outer joint rod 211 are closed, thereby locking the positions of the inner joint rod 212 and the outer joint rod 211.

The connecting member 22 is provided with a third ball-headed rod 227, a concave body 228 and a universal spherical shell 229 that interact with the third ball-headed rod 227. The third ball-headed rod 227 and the concave body 228 are slidably abutted, the third ball-headed rod 227 and the concave body 228 are both located in the universal spherical shell 229, and the third ball-headed rod 227 and the concave body 228 are slidably abutted. When the concave body 228 is integrally connected to the outer joint rod 211, the third ball-headed rod 227 is integrally connected to the inner joint rod 212, and when the concave body 228 is integrally connected to the inner joint rod 212, the third ball-headed rod 227 is integrally connected to the outer joint rod 21. In this embodiment, the concave body 228 is integrally connected to the outer joint rod 211, and the third ball-headed rod 227 is integrally connected to the inner joint rod 212.

It is worth mentioning that, the third ball-headed rod 227 and the concave body 228 form a joint structure, which allows the third ball-headed rod 227 to rotate. Moreover, the third ball-headed rod 227 and the concave body 228 are integrally located inside the universal spherical shell 229, so that the third ball-headed rod 227 rotates more flexibly.

The transpedicular screw 1 is provided with a screw seat 14, a fastening stud (not shown) and a screw body. One end of the screw body penetrates from a lower portion of the screw seat 14 to the outside, the third ball-headed rod 227 and the concave body 228 are both located inside the screw seat 14, and the fastening stud 15 is in threaded connection with an upper end of the screw seat 14.

The transpedicular screw 1 is provided with a screw seat 14, a fastening stud 15 and a screw body (not shown). One end of the screw body penetrates from a lower portion of the screw seat 14 to the outside. The third ball-headed rod 227, the concave body 228 and the universal spherical shell are located inside the screw seat 14, and the fastening stud 15 is in threaded connection with an upper end of the screw seat 14.

Compared with embodiment 1, except for possessing a certain degree of motion freedom, the scoliosis correction device in this embodiment may also extend and retract according to the actual situation of the spine, thereby satisfying the requirements of different situations.

### Embodiment 5

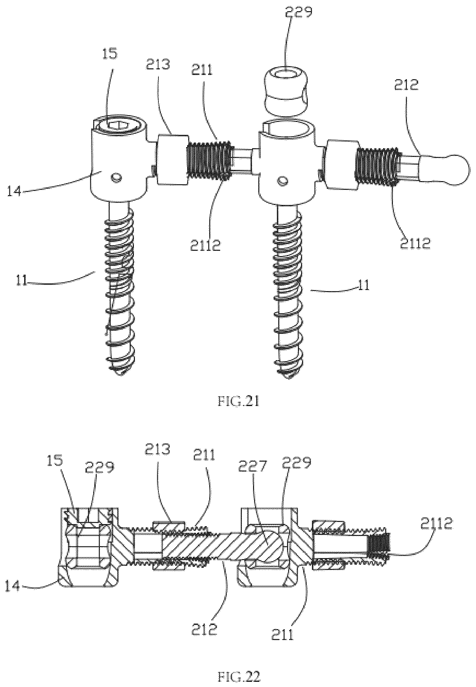

Figures 21-22 show a scoliosis correction device in embodiment 5. In this embodiment, the rod segment 21 is a telescopic rod segment 21. The telescopic rod segment 21 is provided with an outer joint rod 211, an inner joint rod 212 and a locking member 213 for locking the relative positions of the inner joint rod 212 and the outer joint rod 211. The inner joint rod 212 is movably sleeved inside the outer joint rod 211, and the locking member 213 is movably assembled on the outer joint rod 211.

The locking member 213 is a locking knob, the locking knob is sleeved on an outer surface of the outer joint rod 211, and the locking knob is in threaded connection with the outer joint rod 211.

An outer surface of one end of the outer joint rod is integrally connected with an external thread for interacting with the locking knob, wherein one end connected to the external thread is defined as a second working end, and the inner joint rod is telescopically sleeved inside the second working end. A plurality of longitudinal slits are arranged at an end of the second working end, an inner surface of the second working end is integrally connected with teeth 2113, and the the ratchet teeth 2121 are engaged with the teeth 2113.

When adjusting the length of the telescopic rod segment 21, the locking knob is rotated to the outside of the second working end, so that the position of the inner joint rod 212 is adjusted. Subsequently, the locking knob is rotated to the end of the second working end, thereby locking the positions of the inner joint rod 212 and the outer joint rod 211.

It is worth mentioning that, because the end of the second working end is provided with a plurality of slits 2112, when the locking knob is rotated to the outside of the second working end, the inner joint rod 212 is separated from the second working end, allowing the inner joint rod 212 to move in a telescopic manner. After the inner joint rod 212 moves to a proper position, the locking knob is rotated to the end of the second working end, and the slits 2112 of the outer joint rod 211 are closed, thereby locking the positions of the inner joint rod 212 and the outer joint rod 211.

The connecting member 22 is provided with a third ball-headed rod 227 and a universal spherical shell 229, and the third ball-headed rod 227 is slidably abutted against a side wall surface of the universal spherical shell 229. The third ball-headed rod 227 and the concave body 228 are both located in the universal spherical shell 229. The transpedicular screw 1 is provided with a screw seat 14, a fastening stud 15 and a screw body 11.The screw seat 14 is integrally connected to the outer joint rod 211, the screw body penetrates from a lower portion of the screw seat 14 to the outside, and the fastening stud 15 is in threaded connection with an upper end of the screw seat 14. The third ball-headed rod 227 is integrally connected to the inner joint rod 212, the universal spherical shell 229 is integrally assembled inside the screw seat 14, and the third ball-headed rod 227 is movably assembled inside the universal spherical shell 229. When the screw seat 14 is integrally connected to the outer joint rod 211, the third ball-headed rod 227 is integrally connected to the inner joint rod 212, and when the third ball-headed rod 227 is integrally connected to the inner joint rod 212, the screw seat 14 is integrally connected to the outer joint rod 211. In this embodiment, the screw seat 14 is integrally connected to the outer joint rod 211, and the third ball-headed rod 227 is integrally connected to the inner joint rod 212.

It is worth mentioning that, the third ball-headed rod 227 and the universal spherical shell 229 form a joint structure, enabling the third ball-headed rod 227 to rotate such that a certain degree of motion freedom is achieved.

Compared with embodiment 1, except for possessing a certain degree of motion freedom, the scoliosis correction device in this embodiment may also extend and retract according to the actual situation of the spine, thereby satisfying the requirements of different situations.

The above specific embodiments do not constitute a limitation on the scope of protection of the present invention. For those skilled in the art, it should be understood that various modifications, combinations, sub-combinations and substitutions can be made according to design requirements and other factors. Any modification, equivalent substitution and improvement made within the principles of the present invention shall fall in the scope of protection of the present invention.

## Claims

1. A scoliosis correction device, comprising
a plurality of transpedicular screws, and
a flexible orthopedic rod, wherein the plurality of transpedicular screws jointly fix the flexible orthopedic rod, wherein the flexible orthopedic rod comprises a plurality of rod segments and a plurality of connecting members, wherein the plurality of rod segments are movably connected end to end through the plurality of connecting members, and wherein an angle formed by adjacent rod segments is in a range of from 160° to 180°.

2. The scoliosis correction device of claim 1, wherein the connecting members each comprise a hinge housing and two first ball-headed rods, wherein both ends of the hinge housing are respectively provided with a spherical groove or a first spherical cavity matched with the first ball-headed rod, and wherein the hinge housing is connected to two adjacent rod segments through the first ball-headed rods.

3. The scoliosis correction device of claim 2, wherein the plurality of transpedicular screws each comprise a screw body and a screw cap, wherein the screw cap is arranged on the screw body and provided with a through hole sized to fit the hinge housing, and wherein the hinge housing is located in the through hole.

4. The scoliosis correction device of claim 2, wherein the transpedicular screws each comprise a screw body, a screw cap and a second ball-headed rod, wherein the hinge housing is further provided with a second spherical cavity, wherein one end of the second ball-headed rod is provided with a ball-headed matching with the second spherical cavity, while another end of the second ball-headed rod is arranged on the screw cap, and wherein the screw cap is arranged on the screw body.

5. The scoliosis correction device of claim 4, wherein the first ball-headed rod and the screw body are perpendicular to each other.

6. The scoliosis correction device of claim 1, wherein the connecting members each comprise a hinge housing and a first ball-headed rod, wherein the hinge housing is provided with a first spherical cavity, wherein the first ball-headed rod is matched with the first spherical cavity, wherein the first ball-headed rod and the hinge housing are respectively arranged at corresponding ends of two adjacent rod segments, wherein the hinge housing is provided with a limit slot, which is in communication with the first spherical cavity to define a rotation range of the first ball-headed rod, and wherein a rotation angle of the first ball-headed rod defined by the limit slot is in a range of from 0° to 20°.

7. The scoliosis correction device of claim 1, wherein the connecting members each comprise a pin seat and a pin, wherein the pin seat is provided with a pin hole for installing the pin, wherein the pin is located in the pin hole, wherein a plurality of the connecting members are provided between transpedicular screws, and wherein adjacent pins are perpendicular to each other.

8. The scoliosis correction device of claim 1, wherein the transpedicular screws each comprise a screw body, a screw seat and a fastening stud, wherein the screw body is rotatably connected to an inner side of a bottom portion of the screw seat, wherein a threaded section of the screw body is located below the screw seat, wherein through grooves are symmetrically provided on both sides of the screw seat, wherein a thread groove is provided on an inner side of a top portion of the screw seat, wherein the fastening stud is threadedly connected to the thread groove, and wherein the rod segments are sandwiched between the screw body and the fastening stud.

9. The scoliosis correction device of claim 1, wherein the rod segment is a telescopic rod segment, wherein the telescopic rod segment is provided with an outer joint rod, an inner joint rod, and a locking member for locking the relative positions of the inner joint rod and the outer joint rod, wherein the inner joint rod is movably sleeved inside the outer joint rod, and wherein the locking member is movably assembled on the outer joint rod.

10. The scoliosis correction device of claim 9, wherein the surface of the locking member is provided with convex teeth, wherein an outer surface of one end of the inner joint rod is integrally connected with ratchet teeth for interacting with the convex teeth, wherein one end connected to the ratchet teeth is defined as a first working end, wherein the first working end is telescopically sleeved inside the outer joint rod, wherein the locking member is movably assembled on the outer joint rod, wherein the convex teeth are engaged with the ratchet teeth, wherein the outer joint rod is provided with a second through hole for exposing the ratchet teeth and an assembly area for assembling with the locking member, wherein the second through hole is located inside the assembly area, wherein the locking member is movably assembled in the assembly area, and wherein the ratchet teeth extend to the assembly area through the second through hole.

11. The scoliosis correction device of claim 10, wherein the locking member is an elastic pawl, wherein the elastic pawl is movably embedded in an interior of the assembly area, wherein when adjusting the length of the telescopic rod segment, the elastic pawl is compressed, so that the convex teeth are separated from the ratchet teeth and the position of the inner joint rod is adjusted, wherein after the elastic pawl is restored, the convex teeth are engaged with the ratchet teeth, thereby locking the positions of the inner joint rod and the outer joint rod.

12. The scoliosis correction device of claim 10, wherein the locking member is a fine-teeth cam, wherein the fine-teeth cam is rotatably connected to the assembly area through a shaft, wherein when adjusting the length of the telescopic rod segment, the fine-teeth cam is rotated, so that the convex teeth are separated from the ratchet teeth and the position of the inner joint rod is adjusted, wherein after the fine-teeth cam is restored, the convex teeth are engaged with the ratchet teeth, thereby locking the positions of the inner joint rod and the outer joint rod.

13. The scoliosis correction device of claim 9, wherein the locking member is a locking knob, wherein the locking knob is sleeved on an outer surface of the outer j oint rod, and wherein the locking knob is in threaded connection with the outer joint rod.

14. The scoliosis correction device of claim 13, wherein an outer surface of one end of the outer joint rod is integrally connected with an external thread for interacting with the locking knob, wherein one end connected to the external thread is defined as a second working end, wherein the inner joint rod is telescopically sleeved inside the second working end, wherein a plurality of longitudinal slits are arranged at an end of the second working end, wherein an inner surface of the second working end is integrally connected with teeth, wherein the ratchet teeth are engaged with the teeth, wherein when adjusting the length of the telescopic rod segment, the locking knob is rotated to the outside of the second working end such that the position of the inner joint rod is adjusted, wherein when the locking knob is rotated to an end of the second working end, the positions of the inner joint rod and the outer joint rod are locked.

15. The scoliosis correction device of claims 10, wherein the connecting member is provided with a lantern ring and a rotating ring, wherein the rotating ring is rotatably assembled inside the lantern ring, wherein the transpedicular screw penetrates through the rotating ring, wherein the lantern ring is provided with an opening, and wherein the rotating ring is a closed ring.
